# EUROPÄISCHE PATENTSCHRIFT

(11) **EP 3 143 383 B1**
(45) Veröffentlichungstag und Bekanntmachung des Hinweises auf die Patenterteilung: **24.08.2022**
(21) Anmeldenummer: 15726854.1
(22) Anmeldetag: 29.04.2015
(51) Int. Cl.: G01N 21/77, G01N 33/12, B65B 31/00, B65B 57/00, B65B 61/20, B65B 31/02, G01N 31/22

(54) **MESSUNG DER FLUORESZENZ EINES INDIKATORS IN EINER GASDICHTEN VERPACKUNG, DIE PRODUKTE MIT LIMITIERTER LAGERFÄHIGKEIT ENTHÄLT**
DETECTING THE FLUORESCENCE OF AN INDICATOR IN A GAS TIGHT PACKAGE CONTAINING PRODUCTS WITH LIMITED SHELF LIFE
DÉTECTION DE LA FLUORESCENCE D'UN INDICATEUR DANS UN EMBALLAGE ÉTANCHE AU GAZ CONTENANT DES PRODUITS AYANT UNE DURÉE DE CONSERVATION LIMITÉE

(30) Priorität: 14.05.2014 AT 2072014 U
(43) Veröffentlichungstag der Anmeldung: 22.03.2017
(73) Patentinhaber: Tecsense GmbH, 8075 Grambach (AT)
(72) Erfinder: KROTTMAIER, Johannes, 8301 Hart bei Graz (AT); RIBITSCH, Volker, 8010 Graz (AT)
(74) Vertreter: Cunow, Gerda
(86) Internationale Anmeldenummer: PCT/AT2015/000060
(87) Internationale Veröffentlichungsnummer: WO 2015/172166

(56) Entgegenhaltungen:
- EP-A1- 2 256 042
- US-A- 5 407 829
- US-A1- 2007 212 789
- US-A1- 2007 212 789
- US-B2- 7 624 623

## Beschreibung

Die vorliegende Erfindung bezieht sich auf ein Verfahren zur nicht invasiven Messung des Gasgehalts von wenigstens einem Gas, gewählt aus O₂, CO₂, Aminen, Stickoxiden, Schwefelverbindungen in transparenten Verpackungen bestehend aus Kunststofffolien, in welchem ein für den Gasgehalt im Inneren der Verpackung empfindliches Sensormaterial durch darauf auftreffendes Anregungslicht zur Emission eines Fluoreszenz- oder Lumineszenzlichts angeregt wird, das emittierte Fluoreszenz- oder Lumineszenzlicht in einer mit dem Sensor wechselwirkenden Detektionseinrichtung detektiert wird, worauf die verpackten Materialien oder produzierten Materialien in Abhängigkeit von einem in einer mit der Detektionseinrichtung gekoppelten Auswerteeinheit ermittelten Gasgehalt einer Sortierung und gegebenenfalls Zählung unterworfen werden.

Bekannte Messverfahren zum Messen des Gasgehalts von in für Anregungslicht durchlässigen Behältern verpackten Materialien, welche gegenüber einem zu messenden Gas, wie beispielsweise Sauerstoff, Stickoxiden, Ammoniak oder dgl. empfindliche Materialien enthalten oder auch derartige Gase als Zersetzungsprodukte ausbilden, werden derzeit mit nicht kontinuierlich arbeitenden Verfahren in Bezug auf den Gehalt an dem interessierenden Gas überprüft. Diese Verfahren sind einerseits langsam und können andererseits aufgrund der Notwendigkeit, jede Probe einzeln und gesondert zu untersuchen, nur an Stichproben der verpackten Materialien durchgeführt werden, oder es wird eine gesamte Charge mit einer größeren Anzahl an einzelnen Produkten gemeinsam überprüft. Insbesondere bei Lebensmitteln und auf dem pharmazeutischen Bereich ist es erforderlich zu überprüfen, ob Produkte die strengen geforderten Standards erfüllen, und es muss üblicherweise, sollte ein fehlerhaftes Produkt gefunden werden, die gesamte Charge aussortiert werden, da nicht bekannt ist, ob bzw. wie viele andere Proben ebenfalls fehlerhaft sind bzw. über bestimmten Grenzwerten liegende Gasgehalte aufweisen.

Aus der EP 2 256 042 A1 ist eine Verpackungsmaschine mit einer Gaskonzentrationsmesseinreichung bekannt geworden, bei welcher eine Messeinrichtung vorgesehen ist, die einen Messkopf zum Auslesen eines im Inneren der Kammer, im Inneren der Verpackung und/oder im Inneren einer Gasleitung angeordneten Indikators für die Konzentration des Gases mittels elektromagnetischer Strahlung aufweist. Mit einer derartigen Vorrichtung ist es möglich, den Gasgehalt einer Verpackung zu messen, ohne dass eine Probe aus derselben entnommen werden muss.

Der US 2007/212789 A1 ist ein tragbares Messgerät entnehmbar, mit welchem eine im Inneren einer Verpackung angeordnete lumineszierende Verbindung ausgelesen werden kann und somit die Sauerstoffkonzentration in der Verpackung als Funktion der Temperatur gemessen werden kann.

Der US 5 407 829 A ist ein berührungsloser Sensor zum Messen des Gasgehalts im Inneren einer Verpackung entnehmbar.

US2007266773 beschreibt in seinem Stand der Technik mehrere Verfahren, in denen ein Messparameter sensitiv auf Undichtigkeiten in verschlossenen Verpackungen reagiert, wenn Druck auf die Verpackungen angewendet wird.

Ein weiteres wichtiges Kriterium bei der Untersuchung von Materialien in Bezug auf einen gewünschten Gasgehalt besteht darin, dass die Messungen nicht invasiv durchgeführt werden können, da ansonsten die Gefahr der Kontamination mit Schadstoffen besteht bzw. die Verpackungen nach einem gegebenenfalls invasiv geführten Verfahren eine Beschädigung aufweisen, so dass die darin enthaltenen Materialien nach der Testung ebenfalls wiederum verworfen werden müssen. Auch hier besteht das Problem, dass nicht nur die untersuchte Verpackung verworfen werden muss, wenn zu hohe Gehalte an dem zu untersuchenden Gas in der Verpackung enthalten sind, sondern die gesamte Charge, da eine Untersuchung sämtlicher Proben ökonomisch nicht möglich erscheint.

Der hier verwendete Begriff von in Verpackungen enthaltenen "Materialien" ist hierbei nicht auf einen bestimmten Aggregatzustand des Inhalts der Verpackungen beschränkt, sondern umfasst feste, halbfeste und flüssige Materialien ebenso wie in Flüssigkeiten gelöste Gase, insbesondere das zu messende Gase gelöst aufweisende Flüssigkeiten. Weiterhin ist der Begriff Material nicht auf Lebensmittel oder Pharmazeutika beschränkt, sondern kann auch Analyten oder beispielsweise Aufbewahrungs- und/oder Reinigungsflüssigkeiten umfassen.

Die vorliegende Erfindung zielt nun darauf ab, ein nicht invasives Messverfahren zur Verfügung zu stellen, mit welchem es gelingt, sämtliche gleichartigen, in einer Kunststofffolie oder anderen lichtdurchlässigen Materialien verpackten Materialien kontinuierlich zu untersuchen und gezielt fehlerhafte Produkte bzw. Produkte mit einem zu hohem Gehalt an dem zu untersuchenden Gas auszusortieren, ohne dass die gesamte Charge verworfen werden muss und ohne dass bei der Untersuchung eine Beschädigung der Verpackung oder des verpackten Produkts erfolgt.

Zur Lösung dieser Aufgabe ist das erfindungsgemäße Verfahren im Wesentlichen dadurch gekennzeichnet, dass die folgenden Schritte ausgeführt werden:
a) gegebenenfalls Tiefziehen der Verpackung
b) Anbringen eines Sensormaterials auf einer Innenseite einer Abdeckung für die Verpackung oder auf der Innenseite der Verpackung sowie gegebenenfalls Trocknen des Sensormaterials,
c) Einbringen der zu verpackenden Materialien in die Verpackung,
d) gasdichtes Verschließen der Verpackung unter Einbringung einer modifizierten Atmosphäre aus dem zu messenden Gas mit der Abdeckung,
e) Aufbringen der Verpackung auf eine Förder- und Sortiereinrichtung,
f) Anregen des Sensormaterials durch wenigstens eine in definiertem Abstand von der Verpackung angeordnete Anregungslichtquelle,
g) berührungsloses Detektieren der Intensität des von dem Sensormaterial in Abhängigkeit von dem Gasgehalt im Inneren der Verpackung emittierten Fluoreszenz- oder Lumineszenzlichts mittels einer in definiertem Abstand von der Fördereinrichtung angeordneten Detektionseinrichtung, während eines Förderers der Verpackung zu einer Sortiereinrichtung,
h) elektrisches oder elektronisches Umsetzen eines in der Detektionseinrichtung erfassten Messwerts in der Auswerteeinheit,
i) Anlegen eines Drucks auf die Verpackung,
j) sowie neuerliches Durchführen der Schritte f) bis h)
k) Ausgeben eines Bestätigungs- oder Abweisesignal von der Auswerteeinheit, und
l) Weiterführen der Verpackungen zu einer Weiterbearbeitung oder einer Entsorgung in Abhängigkeit von dem durch die Auswerteeinheit errechneten Gasgehalt in der Verpackung.

Indem das erfindungsgemäße Verfahren so geführt wird, das ein Sensormaterial auf einer Innenseite einer Abdeckung für eine Verpackung aufgebracht wird und dieses Sensormaterial, sofern erforderlich, einem Trocknen unterworfen wird, gelingt es, einen Sensor in unmittelbarem Kontakt mit den in einer Verpackung enthaltenen, zu untersuchenden Materialien zur Verfügung zu stellen, welcher Sensor nach einem gasdichten Verschließen der Verpackung direkt auf einer Förder- und Sortiereinrichtung mit Hilfe einer Anregungs- und Detektionseinheit zur Emission von Fluoreszenz- oder Lumineszenzlicht angeregt und das emittierte Fluoreszenz- oder Lumineszenzlicht ausgelesen werden kann. Die Anregungs- und Detektionseinheit weist hierbei in ein und demselben Geräteteil angeordnet wenigstens eine Anregungslichtquelle auf, welche das Sensormaterial in Abhängigkeit von dem Gasgehalt im Inneren der Verpackung zur Emission von Fluoreszenz- oder Lumineszenzlicht anregt, ebenso wie eine mit der wenigstens einen Anregungslichtquelle auf einem gemeinsamen Träger angeordnete Detektionseinrichtung auf, welche in einem definierten Abstand von der Fördereinrichtung angeordnet ist und insbesondere gemeinsam mit der Anregungslichtquelle und einer Mess- bzw. Auswerteeinheit eine Detektions- und Auswerteeinheit ausbildet. Nach Erhalt eines entsprechenden Fluoreszenz- bzw. Lumineszenzsignals und Errechnen eines Messwerts wird dieser Messwert in der Folge in der Detektionseinrichtung elektronisch oder elektrisch umgesetzt und ein Bestätigungs- oder Abweisesignal von der Auswerteeinheit ausgegeben. Im Falle des Erhalts eines Bestätigungssignals, d.h. der Gasgehalt in der Verpackung liegt innerhalb vorgegebener Grenzen, wird von der Förder- und Sortiereinrichtung das in einer Kunststofffolie oder einem anderen lichtdurchlässigen Material verpackte, empfindliche Material einer Weiterverarbeitung, wie beispielsweise einer weiteren Verpackung in großen Gebinden oder für einen unmittelbaren Verbrauch zugeteilt, oder im Falle eines abweisenden Signals einer Entsorgung unmittelbar zugeführt. Mit einem derartigen Verfahren gelingt es, nicht nur jedes einzelne Produkt nicht invasiv zu untersuchen, sondern das Verfahren arbeitet mit einer derartigen Geschwindigkeit, dass die Messung unmittelbar auf einem Förderband erfolgen kann und die Produkte bzw. die verpackten Materialien keinen weiteren gesonderten Detektionsschritten unterworfen werden müssen, wodurch ein besonders effizientes und Zeit sparendes Verfahren zur Ermittlung des Gasgehalts im Inneren einer Verpackung zur Verfügung gestellt werden kann.

Weiterhin wird gemäß der Erfindung, nach einem gasdichten Verschließen der Verpackung ein Druck auf die Abdeckung der Verpackung ausgeübt werden, wodurch im Falle eines Lecks bzw. einer unvollständigen Versiegelung der Verpackung nach Aufheben des Drucks auf die Verpackung Gas/Luft in das Innere der Verpackung einströmt. Somit kann sicher und zuverlässig die Gasdichtheit bei einer nachfolgend ausgeführten zweiten Messung eines Fluoreszenz- oder Lumineszenzsignals bestimmt werden, da ein Messsignal erhalten wird, welches einem von dem ersten Messwert verschiedenen Gasgehalt entspricht. Damit kann eine undichte Verpackung ermittelt werden, ohne das es erforderlich wird, beispielsweise so lange zuzuwarten, bis durch Eindringen durch eine kleine Undichtheit oder durch biologische Zersetzung der im Inneren der Verpackung enthaltenen Produkte eine Verschiebung der Konzentration der einzelnen Restbestände an Gasen in der Verpackung eingetreten ist.

Für eine genaue und insbesondere reversible Messung des Gasgehalts in der Verpackung und insbesondere um zu messen, ob ein Gehalt an zu untersuchenden Gasen einen in der Verpackung zulässigen Grenzwert übersteigt, wird vor der Messung die Verpackung während des Verschließens mit einer modifizierten Atmosphäre aus dem zu messenden Gas beaufschlagt. Bei einer derartigen Verfahrensführung wird sichergestellt, dass einerseits festgestellt werden kann, ob der Verschluss gasdicht ist und andererseits festgestellt werden kann, ob im Inneren der Verpackung enthaltene Produkte, die zu untersuchenden Gase im Zuge ihrer Zersetzung bzw. Zerstörung abgeben, in welchem Fall die Produkte wiederum entsorgt werden müssen. Weiterhin kann, wie dies einer Weiterbildung der Erfindung entspricht, nach einem gasdichten Verschließen der Verpackung ein Druck auf die Abdeckung der Verpackung ausgeübt werden, wodurch im Falle eines Lecks bzw. einer unvollständigen Versiegelung der Verpackung nach Aufheben des Drucks auf die Verpackung Gas/Luft in das Innere der Verpackung einströmt. Somit kann sicher und zuverlässig die Gasdichtheit bei einer nachfolgend ausgeführten zweiten Messung eines Fluoreszenz-oder Lumineszenzsignals bestimmt werden, da ein Messsignal erhalten wird, welches einem von dem ersten Messwert verschiedenen Gasgehalt entspricht. Damit kann eine undichte Verpackung ermittelt werden kann, ohne das es erforderlich wird, beispielsweise so lange zuzuwarten, bis durch Eindringen durch eine kleine Undichtheit oder durch biologische Zersetzung der im Inneren der Verpackung enthaltenen Produkte eine Verschiebung der Konzentration der einzelnen Restbestände an Gasen in der Verpackung eingetreten ist.

Es erübrigt sich festzuhalten, dass der Gasgehalt auch zu einem späteren Zeitpunkt, beispielsweise nach einer gewissen Lagerdauer ausgelesen werden kann, um zu ermitteln, ob eine Leckage vorliegt oder ein Abbau bzw. eine Zersetzung des verpackten Produkts stattfindet bzw. stattgefunden hat.

Um eine kontinuierliche Verfahrensführung sicherzustellen, wird das Verfahren im Wesentlichen so geführt, dass das Messen des Gasgehalts durch serielle Anregung und Detektion des Fluoreszenz- oder Lumineszenzlichts im gleichbleibenden zeitlichen Intervallen von 20 ms bis 80 ms, insbesondere 50 ms durchgeführt wird. Bei einer derartigen Verfahrensführung wird sichergestellt, dass eine Vielzahl von Messwerten generiert wird und weiterhin gewährleistet, dass jede Verpackung sowie die darin enthaltenen bzw. darin eingebrachten Materialien sicher und zuverlässig vermessen werden, worauf durch Auswertung der erhaltenen Messwerte festgestellt werden kann, ob ein verpacktes Produkt bzw. die Verpackung oder das Gebinde selbst den Qualitätsanforderungen entspricht oder nicht entspricht.

Hierbei werden, wie dies einer Weiterbildung der Erfindung entspricht, in der Auswerteeinheit erhaltenen Messwerte absoluten Konzentrationswerten zugeordnet und in Abhängigkeit vom ermittelten Gasgehalt an einer Sortiereinrichtung, insbesondere einer Sortierweiche, das Bestätigungs- oder Abweisesignal ausgelöst. Durch eine derartige Verfahrensführung werden fehlerhafte Produkte mit Sicherheit aussortiert und andererseits werden entsprechende Produkte einer Weiterverarbeitung, wie beispielsweise einer Verpackung in Gebinden oder einem Versand zugeführt.

Eine Vorrichtung, welche für die Durchführung eines derartigen Verfahrens geeignet ist, ist im Wesentlichen dadurch gekennzeichnet, dass wenigstens eine Anregungslichtquelle in definiertem Abstand von dem im Inneren der Verpackung angeordnetem Sensormaterial vorgesehen ist, dass eine mit der wenigstens einen Anregungslichtquelle an einer gemeinsamen Halterung festgelegte Detektionseinheit, insbesondere eine Fotodiode von zur Wechselwirkung mit von dem Sensormaterial emittierten Fluoreszenz- oder Lumineszenzlicht sowie eine elektronische Auswerteeinheit vorgesehen ist und dass die das Sensormaterial enthaltende Verpackung auf einem sich mit gleichbleibender, definierter Geschwindigkeit bewegenden Fördersystem aufgebracht ist. Die wesentlichen Merkmale einer erfindungsgemäßen Vorrichtung bestehen darin, dass das Sensormaterial von der Anregungslichtquelle und der Auswerteeinheit getrennt in direktem Kontakt mit den zu vermessenden Materialien angeordnet ist und das die das Sensormaterial enthaltende Verpackung auf ein sich mit gleichbleibend definierter Geschwindigkeit bewegenden Fördersystem aufgebracht wird und in Bezug auf die Anregungs- und Detektionseinheit bewegbar angeordnet ist. Hierbei wird das Verfahren im Wesentlichen so geführt, dass Anregungslicht von der wenigstens einen Anregungslichtquelle auf das Sensormaterial auftritt, worauf dieses während des Vorbeiführens zur Emission von Fluoreszenz- oder Lumineszenzlicht angeregt wird und gleichzeitig das emittierte Fluoreszenz- oder Lumineszenzsignal in der Auswerteeinheit detektiert und in ein elektrisches bzw. elektronisches Signal umgewandelt wird, welches der Auswerteeinheit zugeführt wird und in der Folge der Gehalt an dem in der Verpackung enthaltenen schädlichen Gas berührungslos und zeitnah erfasst wird.

Für sichere und gleichbleibende und insbesondere reproduzierbare Messergebnisse ist die erfindungsgemäße Vorrichtung im Wesentlichen so ausgebildet, dass zwei LEDs als Anregungslichtquellen vorgesehen sind. Mit einer derartigen Vorrichtung wird sichergestellt, dass Anregungslicht auf jeden einzelnen Sensorspot auftrifft und jede einzelne zu untersuchende Verpackung mit Anregungslicht bestrahlt wird, um den Gasgehalt im Inneren jeder einzelnen Verpackung messen zu können.

Indem, wie dies einer Weiterbildung der vorliegenden Erfindung entspricht, die Vorrichtung so ausgebildet ist, dass eine Mehrzahl Linsen und/oder optischen Filtern der Detektionseinheit vorgeschalten ist, wird eine Bündelung des Fluoreszenz- bzw. Lumineszenzlichts in Richtung auf die Detektionseinheit erreicht, wodurch ein weiter vereinheitlichtes bzw. verbessertes Messergebnis erhalten werden kann.

Um gleichzeitig sicherzustellen, dass eine Vielzahl von in gleichen Zeitabständen ausgesandten Lichtpulsen auf die Verpackungen auftrifft, ist die erfindungsgemäße Vorrichtung so weitergebildet, dass wenigstens ein Pulsgeber vorgesehen ist, um periodisch Anregungslicht auszusenden. Mit einer derartigen Vorrichtung werden Lichtpulse in definierten Zeitabständen, insbesondere zwischen 20 ms und 80 ms, vorzugsweise 50 ms ausgesandt, so dass mit Sicherheit jeder einzelne Sensorspot mit Anregungslicht beaufschlagt wird und wenigstens ein Fluoreszenz- oder Lumineszenzereignis durch das Anregungslicht an jedem einzelnen Sensorspot angeregt wird, um zuverlässig jede einzelne Verpackung in Bezug auf den Gasgehalt in ihrem Inneren untersuchen zu können.

Indem, wie dies einer Weiterbildung der Erfindung entspricht, die Auswerteeinheit mit einer Sortier- und Zählvorrichtung für die Verpackungen gekoppelt ist, gelingt es weiterhin, die vermessenen Verpackungen je nach ihrem Gasgehalt auszusortieren, wobei die Verpackungen, welche einen Gasgehalt in einem zulässigen Bereich enthalten, einer Weiterverarbeitung, wie z.B. ein Verpacken in Gebinde, zugeführt werden und Verpackungen, welche einen zu hohen bzw. zu niedrigen Gasgehalt aufweisen, aussortiert werden. In gleicher Weise kann die Auswerteeinheit eine Zählvorrichtung enthalten, mit welcher nicht nur die Anzahl der Verpackungen insgesamt ermittelt werden kann, sondern auch die Zahl der aussortierten bzw. zulässigen Verpackungen ermittelt werden kann.

Die Erfindung wird nachfolgernd anhand eines Ausführungsbeispiels sowie einer Zeichnung näher erläutert. In dieser zeigt:
Fig. 1 eine schematische Darstellung eines Messsystems gemäß der Erfindung, und
Fig. 2 ein Diagramm, welches die Messergebnisse für den Gehalt eines zu untersuchenden Gases in einer Probe zeigt.

### Beispiel 1:

### Sauerstoffgehalt in Wurst enthaltenden Verpackungen

Eine als Deckfolie für eine Wurstverpackung gedachte Kunststofffolie wird auf ihrer nach einer Verpackung des Wurstprodukts eine Innenseite darstellenden Oberfläche mittels Spucken mit wenigstens einen diskreten kleinen Punkt eines Sensormaterials beschichtet. Hierzu werden mit einem getakteten Farbspuckers (Drop on demand) auf die die Innenseite der eine Abdeckung ausbildende Folie Sensorpunkte berührungslos aufgebracht. Die Punkte werden einem Trocknen für zwei Sekunden im Warmluftstrom unterworfen, während die Deckfolie weiter zu der Verpackungsvorrichtung gefördert wird, wo ein Lebensmittel, beispielsweise Wurst, in den unteren Teil der Verpackung eingebracht wird und anschließend in die Verpackung eine modifizierte Atmosphäre aus dem zu messenden Gas, im vorliegenden Fall O₂ eingebracht und heiß versiegelt wird. Eine derartig hergestellte Verpackung wird in einem ersten Schritt unmittelbar nach der Verpackung auf einer Fördervorrichtung abgelegt und während des Förderns einer berührungslosen Messung des Sauerstoffgehalts im Inneren der Verpackung unterworfen. Hierzu wird von einer in Abstand von der Verpackung angeordneten Anregungslichtquelle alle 50 ms ein Lichtpuls ausgesandt, welches Anregungslicht das im Inneren der Verpackung angeordnete Sensormaterial in Abhängigkeit von dem im Inneren der Verpackung vorherrschenden Sauerstoffgehalt zur Abgabe von Fluoreszenzlicht anregt. Das Fluoreszenzlicht wird mittels einer mit den Anregungslichtquellen auf ein und demselben Träger angeordneten Mehrzahl von Linsen bzw. Spiegeln auf eine Photodiode fokussiert. An der Photodiode wird ein elektrischer Impuls erzeugt, welcher in einer mit der Photodiode verbundenen Elektronik digital in einen Messwert betreffend die Sauerstoffkonzentration im Inneren Verpackung verarbeitet wird.

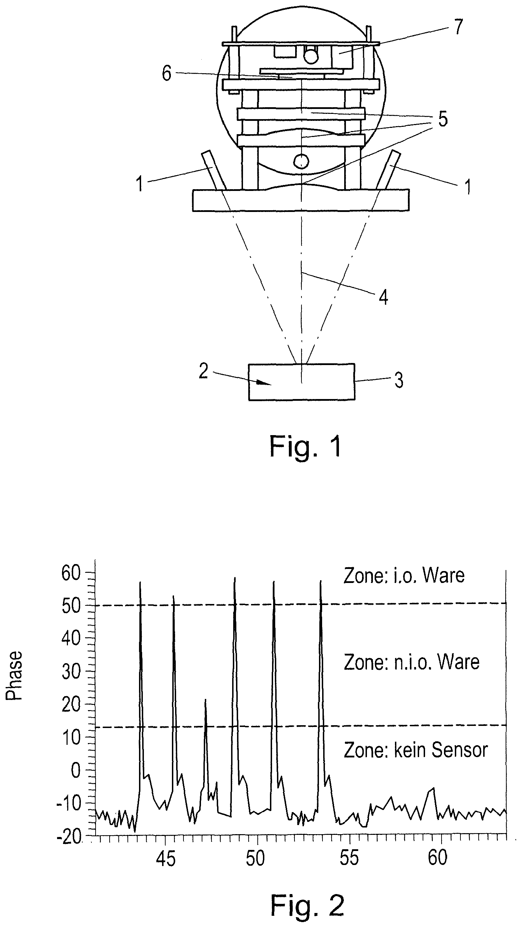

Eine derartige Vorrichtung ist in Fig. 1 dargestellt. Bei der Vorrichtung wird durch die Anregungslichtquelle 1 gepulstes Anregungslicht auf den im Inneren der schematisch dargestellten Verpackung 3 angeordneten Sensor 2 auftreffen gelassen, wobei von dem Sensor 2 emittiertes Fluoreszenzlicht, welches schematisch mit 4 angedeutet ist, über eine Mehrzahl von Linsen oder Spiegeln 5 auf eine Detektionseinrichtung 6 auftrifft. An der Detektionseinrichtung 6 wird das Fluoreszenzlicht in ein elektronisches Signal umgewandelt, welches Signal in der Auswerteeinheit 7 in Bezug auf seine Intensität ausgewertet wird und durch Vergleichen mit während eines Kalibrierverfahrens erhaltenen Vergleichswerten aus der Intensität des Signals eine absolute Konzentration des zu messenden Sauerstoffgehalts in der Probe errechnet wird.

Die Auswerteeinheit 7 gibt hierbei während des Durchlaufs einer Mehrzahl von Verpackungen 3 unter der Messeinheit, jedes Mal wenn ein Sensor 2 mit dem Anregungslicht 1 zur Abgabe von Fluoreszenzlicht angeregt wurde, ein Messsignal aus. Ein schematisches Diagramm der Messsignale ist in Fig. 2 dargestellt. Hierbei wird der in der Detektionseinrichtung ermittelte Messwert einem absoluten Messwert zugeordnet, indem die Sensoren, welche für die Verpackungen 3 eingebracht wurden, zuvor kalibriert wurden, indem sie mit verschiedenen Gaskonzentrationen beaufschlagt und vermessen wurden und die Messwerte den bestimmten Gaskonzentrationen zugeordnet wurden. Hierbei wird in Bezug auf die Sauerstoffkonzentration in normaler Luft von etwa 20 % O₂ abgestellt und eine Kalibrierung zwischen 0,1 % O₂ und 20 % O₂ vorgenommen, da im Inneren der Verpackungen ein O₂-Gehalt von 0,2 % vorliegen soll. Bei dem mit mit einer derartigen Auswerteeinheit erhaltenen Diagramme sind zwischen einer Phase von -20 und +13 ein Rauschen abgebildet bzw. der Bereich dargestellt ist, in welchem kein Sensor vorhanden ist, eine Phase zwischen 13 und 50 den Bereich darstellt, in welchem der Sauerstoffgehalt im Inneren der Probe zu hoch ist und die Proben verworfen werden müssen und Werte, welche eine Phase von über 50 aufweisen, jene Werte sind, die einer zulässigen Sauerstoffkonzentration im Inneren der Verpackung entsprechen und somit Waren definieren, welche in Ordnung sind. Aus Fig. 2 ist ersichtlich, dass eine Probe einen zu hohen Sauerstoffgehalt im Inneren der Verpackung aufwies, welche nachfolgend unmittelbar durch die Vorrichtung aussortiert und verworfen wurde.

### Beispiel 2:

Sauerstoffgehalt in einer Reinigungs- und Aufbewahrungslösung für Kontaktlinsen, welche in einer Verpackung gemäß der Erfindung enthalten ist

Eine Deckfolie für einen als Aufbewahrungsbehälter von Kontaktlinsen gedachten Glasbehälter wird auf ihrer nach einem Verpacken der Kontaktlinsen bzw. nach einem Befüllen mit der Reinigungs- und Aufbewahrungslösung der Befüllung zugewandten Oberfläche mit jeweils einem diskreten Punkt eines Sensormaterials beschichtet. Die Beschichtung erfolgt mittels Siebdruck und die ausgebildeten Sensorpunkte werden einem Trocknen im Warmluftstrom unterworfen, bevor sie auf einen Aufbewahrungsbehälter für Kontaktlinsen, welcher neben der Kontaktlinse auch die Aufbewahrungsflüssigkeit enthält, aufgebracht werden.

Die Aufbewahrungsflüssigkeit ist in diesem Fall mit dem zu messenden Gas gesättigt, im vorliegenden Fall Sauerstoff. Sie weist einen bekannten Sauerstoffgehalt auf und die Messung des Sauerstoffgehaltes erfolgt in Bezug auf den Umgebungssauerstoffgehalt, wobei Unterschiede in den O₂-Gehalten zwischen dem Inneren des Behälters und der Außenseite von weniger als 5 % ausreichend sind, um zu bestätigen, dass eine Probe, die die vorgegebenen Kriterien erfüllt, d.h. der Sauerstoffgehalt im Inneren des Aufbewahrungsbehälters insbesondere der gelöste Sauerstoffgehalt einen gewissen Grenzwert von 5 % nicht übersteigt.

Derartig befüllte und verschlossene Glasbehälter werden in der Folge auf eine Fördervorrichtung gestellt und jede einzelne Flasche wird einem berührungslosen Messen, wie dies in Beispiel 1 beschrieben wurde, unterworfen und die Ergebnisse aufgezeichnet. Die Anregungslichtquelle sendet hierbei das Anregungslicht gepulst aus, wobei die Pulsweite 40 ms beträgt, um sicherzustellen, dass sämtliche Proben auch vermessen werden. Das Messverfahren selbst erfolgt exakt wie in Beispiel 1 beschrieben. Ein erhaltenes Messdiagramm zeigt, im Fall des Versuchs von Beispiel 2 an, ob die Sauerstoffkonzentration in der Reinigungs- und Aufbewahrungsflüssigkeit für Kontaktlinsen zu hoch oder zu niedrig oder in dem gewünschten Bereich liegt.

Um absolute Sicherheit zu erhalten, dass die hergestellte Verpackung tatsächlich gasdicht ist, kann das Verfahren auch so geführt werden, dass unmittelbar nach der Verpackung eine erste Messung, wie oben beschrieben, durchgeführt wird, die Probe anschließend mit Druck beaufschlagt wird und danach eine zweite Messung durchgeführt wird. Wenn die Versiegelung der Verpackung nicht rundum dicht ist, wird nach dem Aufbringen von Druck jedenfalls Sauerstoff in das Innere der Verpackung aufgenommen, worauf der zweite Messwert dem ersten Messwert nicht mehr entspricht und klargestellt ist, dass die Verpackung undicht ist, worauf das Produkt unmittelbar aussortiert werden kann.

Es erübrigt sich festzuhalten, dass es nicht unbedingt erforderlich ist, die Sensoren zu kalibrieren sondern, dass auch die ausschließliche Durchführung von Relativmessungen die gewünschten Ergebnisse, ob ein Inhalt der Verpackung eine sich in einen zulässigen Bereich befindliche Gaskombinationen aufweist, liefert.

Dieselbe Anordnung kann beispielsweise auch für die Messung des Gasgehalts in medizinischen Artikeln, Pharmazeutika und Elektronikbauteilen angewandt werden.

### Beispiel 3:

Messung des Sauerstoffgehalts in einem mit Argon befüllten Zwischenraums von zweischeibigen Isolierglasfenstern

Dabei wird das Sensormaterial auf die dem Zwischenraum zwischen den Glasscheiben zugewandte Seite einer Glasscheibe geklebt oder gedruckt und wie in Beispiel 1 oder 2 beschrieben, von außen angeregt und ausgelesen. Dies dient zur Kontrolle, ob der Füllgrad mit Edelgas ausreichend groß ist.

## Patentansprüche

1. Verfahren zur nicht invasiven Messung des Gasgehalts von wenigstens einem Gas, gewählt aus O₂, CO₂, Aminen, Stickoxiden, Schwefelverbindungen in transparenten Verpackungen bestehend aus Kunststofffolien, in welchem ein für den Gasgehalt im Inneren der Verpackung (3) empfindlicher Sensor (2) durch darauf auftreffendes Anregungslicht zur Emission eines Fluoreszenz- oder Lumineszenzlichts angeregt wird, das emittierte Fluoreszenz- oder Lumineszenzlicht in einer mit dem Sensor (2) wechselwirkenden Detektionseinrichtung (6) detektiert wird, worauf die verpackten Materialien oder produzierten Materialien in Abhängigkeit von einem in einer mit der Detektionseinrichtung (6) gekoppelten Auswerteeinheit (7) ermittelten Gasgehalt einer Sortierung und gegebenenfalls Zählung unterworfen werden, wobei die folgenden Schritte ausgeführt werden:
a) gegebenenfalls Tiefziehen der Verpackung
b) Anbringen eines Sensormaterials auf einer Innenseite einer Abdeckung für die Verpackung oder auf der Innenseite der Verpackung sowie gegebenenfalls Trocknen des Sensormaterials,
c) Einbringen der zu verpackenden Materialien in die Verpackung,
d) gasdichtes Verschließen der Verpackung unter Einbringung einer modifizierten Atmosphäre aus dem zu messenden Gas mit der Abdeckung,
e) Aufbringen der Verpackung auf eine Förder- und Sortiereinrichtung,
f) Anregen des Sensormaterials durch wenigstens eine in definiertem Abstand von der Verpackung angeordnete Anregungslichtquelle (1),
g) berührungsloses Detektieren der Intensität des von dem Sensormaterial in Abhängigkeit von dem Gasgehalt im Inneren der Verpackung emittierten Fluoreszenz- oder Lumineszenzlichts mittels einer in definiertem Abstand von der Fördereinrichtung angeordneten Detektionseinrichtung, während eines Förderns der Verpackung zu einer Sortiereinrichtung.
h) elektrisches oder elektronisches Umsetzen eines in der Detektionseinrichtung erfassten Messwerts in der Auswerteeinheit,
i) Anlegen eines Drucks auf die Verpackung,
j) sowie neuerliches Durchführen der Schritte f) bis h)
k) Ausgeben eines Bestätigungs- oder Abweisesignals von der Auswerteeinheit, und
l) Weiterführen der Verpackungen zu einer Weiterbearbeitung oder einer Entsorgung in Abhängigkeit von dem durch die Auswerteeinheit errechneten Gasgehalt in der Verpackung.

2. Verfahren nach Anspruch 1, **dadurch gekennzeichnet, dass** das Sensormaterial durch Tampondruck, Siebdruck, Offsetdruck, Tiefdruck, Brushen oder Spucken oder Etikettieren auf die Abdeckung der Verpackung aufgebracht wird.

3. Verfahren nach Anspruch 1 oder 2, **dadurch gekennzeichnet, dass** ein auf O₂, CO₂, NH₃, Amine oder NO₂ ansprechendes Sensormaterial auf die Abdeckung der Verpackung aufgebracht wird.

4. Verfahren nach Anspruch 1, 2 oder 3, **dadurch gekennzeichnet, dass** die Verpackung während des Verschließens evakuiert wird.

5. Verfahren nach einem der Ansprüche 1 bis 4, **dadurch gekennzeichnet, dass** das Messen des Gasgehalts durch serielle Anregung und Detektion des Fluoreszenz- oder Lumineszenzlichts in gleichbleibenden zeitlichen Intervallen von 20 ms bis 80 ms, insbesondere 50 ms, durchgeführt wird.

6. Verfahren nach einem der Ansprüche 1 bis 5, **dadurch gekennzeichnet, dass** in der Auswerteeinheit erhaltene Messwerte absoluten Konzentrationswerten zugeordnet werden und dass in Abhängigkeit vom ermittelten Gasgehalt an einer Sortiereinrichtung, insbesondere einer Sortierweiche, das Bestätigungs- oder Abweisesignal ausgelöst wird.

## Claims

1. A method for the non-invasive measurement of the gas content of at least one gas selected from O₂, CO₂, amines, nitrogen oxides, sulfur compounds, in transparent packages made of plastic films, in which a sensor sensitive to the gas content in the interior of the package is excited by excitation light incident thereon to emit a fluorescent or luminescent light, the emitted fluorescent or luminescent light is detected in a detection device interacting with the sensor, whereupon the packaged materials or produced materials are subjected to sorting, and optionally counting, as a function of the gas content determined by an evaluation unit coupled to the detection device, **characterized in that** the following steps are performed:
a) optionally deep-drawing the package;
b) applying a sensor material to the inner side of a cover for the package, or to the inner side of the package, and optionally drying the sensor material;
c) introducing into the package the materials to be packaged;
d) gas-tightly closing the package by the cover while introducing a modified atmosphere of the gas to be measured;
e) placing the package on a conveying and sorting device;
f) exciting the sensor material by at least one excitation light source arranged at a defined distance from the package;
g) detecting, in a contactless manner, the intensity of the fluorescent or luminescent light emitted by the sensor material as a function of the gas content in the interior of the package, by a detection device arranged at a defined distance from the conveying device during conveying of the package to the sorting device;
h) electrically or electronically converting in the evaluation unit a measurement value detected in the detection device;
i) applying a pressure to the package;
j) performing steps f) to h) again;
k) outputting a confirmation signal or a rejection signal by the evaluation unit; and
l) forwarding the package to further processing or disposal as a function of the gas content in the package calculated by the evaluation unit.

2. A method according to claim 1, **characterized in that** the sensor material is applied to the cover of the package by pad printing, screen printing, offset printing, gravure printing, brushing or sputtering, or labeling.

3. A method according to claim 1 or 2, **characterized in that** a sensor material reacting to O₂, CO₂, NH₃, amines or NO₂ is applied to the cover of the package.

4. A method according to claim 1, 2 or 3, **characterized in that** the package is evacuated during closure.

5. A method according to any one of claims 1 to 4, **characterized in that** the measurement of the gas content is performed by the serial excitation and detection of the fluorescent or luminescent light at constant time intervals of 20 ms to 80 ms, in particular 50 ms.

6. A method according to any one of claims 1 to 5, **characterized in that** measurement values obtained in the evaluation unit are assigned to absolute concentration values, and that the confirmation signal or the rejection signal is triggered as a function of the determined gas content on a sorting device, in particular a sorting shunt.

## Revendications

1. Procédé pour la mesure non invasive de la teneur en gaz d'au moins un gaz, choisi parmi O₂, CO₂, aminés, oxydes d'azote, composés de soufre dans des emballages transparents composés de films de matière plastique, dans lequel un capteur (2) sensible à la teneur en gaz à l'intérieur de l'emballage (3) est excité par une lumière d'excitation arrivant sur lui pour l'émission d'une lumière fluorescente ou luminescente, la lumière fluorescente ou luminescente émise est détectée dans un dispositif de détection (6) interagissant avec le capteur (2), après quoi les matériaux emballés ou matériaux produits sont soumis à un tri et éventuellement à un comptage en fonction d'une teneur en gaz déterminée dans une unité d'évaluation (7) couplée au dispositif de détection (6), dans lequel les étapes suivantes sont réalisées :
a) éventuellement emboutissage de l'emballage
b) installation d'un matériau capteur sur un côté intérieur d'un recouvrement pour l'emballage ou sur le côté intérieur de l'emballage ainsi que séchage éventuellement du matériau capteur,
c) introduction des matériaux à emballer dans l'emballage,
d) fermeture étanche au gaz de l'emballage avec introduction d'une atmosphère modifiée à partir du gaz à mesurer avec le recouvrement,
e) pose de l'emballage sur un dispositif de transport et de tri,
f) excitation du matériau capteur par au moins une source de lumière d'excitation (1) disposée à une distance définie de l'emballage,
g) détection sans contact de l'intensité de la lumière fluorescente ou luminescente émise par le matériau capteur en fonction de la teneur en gaz à l'intérieur de l'emballage à l'aide d'un dispositif de détection disposé à une distance définie du dispositif de transport, pendant un transport de l'emballage jusqu'à un dispositif de tri,
h) conversion électrique ou électronique d'une valeur de mesure relevée dans le dispositif de détection dans l'unité d'évaluation,
i) application d'une pression à l'emballage,
j) ainsi que réalisation, à nouveau, des étapes f) à h)
k) émission d'un signal de confirmation ou de refus par l'unité d'évaluation, et
l) poursuite de l'amenée des emballages jusqu'à un traitement ultérieur ou à une élimination en fonction de la teneur en gaz calculée dans l'unité d'évaluation dans l'emballage.

2. Procédé selon la revendication 1, **caractérisé en ce que** le matériau capteur est appliqué sur le recouvrement de l'emballage par impression au tampon, sérigraphie, impression offset, impression hélio, impression à la brosse ou jet d'encre ou étiquetage.

3. Procédé selon la revendication 1 ou 2, **caractérisé en ce qu'**un matériau capteur réagissant à O₂, CO₂, NH₃, amines ou NO₂ est appliqué sur le recouvrement de l'emballage.

4. Procédé selon la revendication 1, 2 ou 3, **caractérisé en ce que** l'on fait le vide dans l'emballage pendant la fermeture.

5. Procédé selon l'une des revendications 1 à 4, **caractérisé en ce que** la mesure de la teneur en gaz est réalisée par excitation et détection sérielles de la lumière fluorescente ou luminescente à des intervalles de temps constants de 20 ms à 80 ms, en particulier de 50 ms.

6. Procédé selon l'une des revendications 1 à 5, **caractérisé en ce que** les valeurs de mesure obtenues dans l'unité d'évaluation sont affectées à des valeurs de concentration absolues et **en ce qu'**en fonction de la teneur en gaz déterminée, le signal de confirmation ou de refus est déclenché à un dispositif de tri, en particulier un aiguillage de tri.
